# EUROPEAN PATENT SPECIFICATION

(11) **EP 3 431 983 B1**
(45) Date of publication and mention of the grant of the patent: **23.04.2025**
(21) Application number: 16894358.7
(22) Date of filing: 15.03.2016
(51) Int. Cl.: G01N 30/06, C08F 236/10, G01N 30/88

(54) **ANALYSIS METHOD FOR A COPOLYMER OF A CONJUGATED DIENE COMPOUND WITH AROMATIC VINYL COMPOUND**
ANALYSEVERFAHREN FÜR EIN COPOLYMER EINER KONJUGIERTEN DIENVERBINDUNG MIT AROMATISCHER VINYLVERBINDUNG
PROCÉDÉ D'ANALYSE DESTINÉ À UN COPOLYMÈRE D'UN COMPOSÉ DIÈNE CONJUGUÉ À UN COMPOSÉ VINYLIQUE AROMATIQUE

(43) Date of publication of application: 23.01.2019
(73) Proprietor: Shimadzu Corporation, Kyoto-shi, Kyoto 604-8511 (JP); The Yokohama Rubber Co., Ltd., Hiratsuka-shi, Kanagawa 254-8601 (JP)
(72) Inventor: YAMAKI, Satoshi, Kyoto-shi Kyoto 604-8511 (JP); IIDA, Tetsuo, Kyoto-shi Kyoto 604-8511 (JP); SEKINE, Yuko, Hiratsuka-shi Kanagawa 254-8601 (JP); ASHIURA, Makoto, Hiratsuka-shi Kanagawa 254-8601 (JP); YATSUYANAGI, Fumito, Hiratsuka-shi Kanagawa 254-8601 (JP)
(74) Representative: Mewburn Ellis LLP
(86) International application number: PCT/JP2016/058207
(87) International publication number: WO 2017/158740

(56) References cited:
- WO-A1-2013/115010
- JP-A- 2012 208 081
- JP-A- H09 316 132
- US-A1- 2003 070 988
- NOBUKO KATO ET AL: "Sequence Characterization of Styrene-Butadiene Rubber by Ozonolysis- Liquid chromatography/Mass spectrometry", INTERNATIONAL RUBBER CONFERENCE 95 KOBE,, 1 January 1995 (1995-01-01), pages 299 - 302, XP009508870
- ANTON GINZBURG ET AL: "Characterization of polyolefins by comprehensive high-temperature two-dimensional liquid chromatography (HT 2D-LC)", EUROPEAN POLYMER JOURNAL, PERGAMON PRESS LTD. OXFORD, GB, vol. 47, no. 3, 24 November 2010 (2010-11-24), pages 319 - 329, XP028145596, ISSN: 0014-3057, [retrieved on 20101201], DOI: 10.1016/J.EURPOLYMJ.2010.11.016
- ANJA BAUMGAERTEL ET AL: "Recent developments in the detailed characterization of polymers by multidimensional chromatography", JOURNAL OF CHROMATOGRAPHY A, ELSEVIER, AMSTERDAM, NL, vol. 1240, 10 March 2012 (2012-03-10), pages 1 - 20, XP028482007, ISSN: 0021-9673, [retrieved on 20120323], DOI: 10.1016/J.CHROMA.2012.03.038
- NOBUKO KATO ET AL.: "Sequence Characterization of Styrene-Butadiene Rubber by Ozonolysis- Liquid chromatography/Mass spectrometry", INTERNATIONAL RUBBER CONFERENCE 95 KOBE, 1995, pages 299 - 302, XP009508870
- NOBUKO KATO: "Application of Rubber Analysis to 'MONODUKURI (Products Manufacturing)", JOURNAL OF THE SOCIETY OF RUBBER INDUSTRY, vol. 82, no. 4, 2009, pages 142 - 149, XP055420440

## Description

### TECHNICAL FIELD

The present invention relates to a method for analyzing a copolymer of a conjugated diene monomer with an aromatic vinyl monomer, and in particular, to a method for analyzing styrene-butadiene rubber (SBR) to be used as a material for industrial products such as tires for automobiles or vibration-proof rubber.

### BACKGROUND ART

Tires for automobiles (pneumatic tires for automobiles) are constituted by parts such as a carcass, an inner liner, a bead wire, and a tread compound, and materials suitable for the functions of the parts are selected as materials of the parts. In particular, the tread compound positioned on the outermost periphery of the tire is involved in running performance of automobiles such as braking performance or rolling resistance of the tire, so that the selection of suitable materials is important in terms of improving the running performance of automobiles.

The material for the tread compound usually contains a polymer, a filler and a softening agent. Among these, the properties of the polymer which accounts for approximately 40% of the whole materials are said to be involved in the running performance of the tire. A styrene-butadiene rubber (SBR) which is a copolymer of styrene with 1,3-butadiene is excellent in heat resistance, wear resistance, mechanical strength, and other properties, and is therefore widely used as a material for automobile tire tread compounds. The SBR has a characteristic chain structure in which styrene, cis-1,4-butadiene, trans-1,4-butadiene, and vinyl are linearly linked in a large number, so that a method for properly evaluating the performance of the tread compound by analyzing the SBR chain structure is sought.

Examples of a method for analyzing a structure of an organic compound include infrared spectroscopy, nuclear magnetic resonance (NMR) spectroscopy, and chromatography. For analyzing the SBR chain structure, any of these methods disadvantageously provides insufficient information, or conversely, provides excessively complex information, which is time-consuming for analysis. For example, the infrared spectroscopy can conveniently measure amounts of styrene, vinyl, cis-butadiene, and trans-butadiene contained in the SBR, but can acquire only information in monomer units. The NMR spectroscopy can provide information about the structure at an atomic level, but the information is excessively complex, so that analysis of the SBR chain structure is time-consuming. Further, as a technique for separating a pyrolyzate obtained by pyrolyzing the SBR, pyrolysis gas chromatography (GC) may be used. This technique, however, can only provide information about the amount ratio of styrene to butadiene that constitute the SBR.

### CITATION LIST

### NON PATENT LITERATURE

Non-Patent Literature 1: Tanaka et al., Preprints for the Society of Polymer Science, vol. 29, No. 9, p. 2055
Non-Patent Literature 2: Y. Tanaka, H. Sato and Y. Nakafumati, Polymer, 22, 1721 (1981)
Non-Patent Literature 3: Y. Tanaka, H. Sato, Y. Nakafumati and Y. Kashiwazaki, Macromolecules, 16, 1925 (1983)
Non-Patent Literature 4: N. Kato, M. Harada, N. Ohshima, S. Kurihara and H. Kuramasu, International Rubber Conference 95 Kobe (1995)
Non-Patent Literature 5: A. Baumgaertel, E Altuntas and U. Schubert, Journal of Chromatography A, 1240, 1 (2021)

### PATENT LITERATURE

Patent Literature 1: US 2003/070988

### SUMMARY OF INVENTION

### TECHNICAL PROBLEM

A glass transition point (Tg) is one of indexes representing performance of rubber. The lower the glass transition point of a rubber is, the more the degradation of the performance is suppressed in a low temperature region, which can provide a tire having excellent cold resistance. Since the glass transition point of an SBR is known to be determined depending on the amounts of styrene and vinyl, there has been proposed a method in which the SBR is decomposed into a chain component composed of a component derived from styrene, a chain component composed of a component derived from vinyl, and a chain component composed of a component derived from styrene and a component derived from vinyl by ozonolysis reaction, and these chain components are analyzed by gel permeation chromatography (GPC) (Non-Patent Literature 1). In this method, however, there is a problem that although the chain component derived from styrene can be separated by the difference in the number of chains, the chain component derived from vinyl has a poor separation ability.

Non-Patent Literature 4 describes the analysis of styrene-vinyl sequences in SBR by ozonolysis-LC/MS. The chain components were separated by reverse phase LC. Non-Patent Literature 5 describes the characterization of polymers by multidimensional chromatography. Patent Literature 1 describes methods and apparatus for characterizing libraries of polymers samples using in a comprehensive, directly-coupled multi-dimensional liquid chromatography system.

The SBR is described herein as an example, but a copolymer of a conjugated diene compound with an aromatic vinyl compound has also a similar problem to the SBR.

An object of the present invention is to analyze all components obtained by ozonolysis of a copolymer of a conjugated diene compound with an aromatic vinyl compound.

### SOLUTION TO PROBLEM

The present invention that has been made to solve the above-mentioned problem is a method for analyzing a copolymer, the method including:
a) subjecting a copolymer of a conjugated diene compound with an aromatic vinyl compound to ozonolysis to produce a plurality of kinds of polyhydric alcohol compounds;
b) separating a sample solution containing the plurality of kinds of polyhydric alcohol compounds with a comprehensive two-dimensional liquid chromatograph equipped with a first column and a second column; and
c) detecting the separated sample with a mass spectrometer,
   wherein:
   the detector is a mass spectrometer; and
   the first column and the second column have different polarities or different separation modes; and wherein the method comprises:
      referring to a calibration curve representing a relationship between a concentration of each kind of the polyhydric alcohol compounds and an ion intensity of the detector; and
      calculating a concentration of a polyhydric alcohol compound from the ion intensity with respect to the polyhydric alcohol compounds in the sample solution detected with the detector.

When the copolymer of the conjugated diene compound with the aromatic vinyl compound is subjected to ozonolysis, a carbon-carbon double bond derived from the conjugated diene compound among carbon-carbon double bonds of the copolymer is cleaved to produce an ozonide, and the ozonide is ring-opened by reduction reaction to produce a polyhydric alcohol compound. At this time, polyhydric alcohol compounds with various chain structures can be obtained in which the number or kind of linking monomers varies depending on the structure of the moiety sandwiched between two carbon-carbon double bonds of the copolymer. For example, when a styrene-butadiene copolymer is subjected to ozonolysis, the moiety polymerized at the 1,4- bond is cleaved. Furthermore, a vinyl group in a side chain is oxidized to be a hydroxymethyl group. As a result, in the styrene-butadiene copolymer, a repeating unit which is sandwiched by adjacent two butadiene units polymerized at the 1,4-bonds is produced as an ozonolysis component. For example, when the moiety sandwiched between two butadiene units that have been polymerized at the 1,4-bonds in the main chain is subjected to ozonolysis, a polyhydric alcohol compound with a chain structure composed of styrene units, a polyhydric alcohol compound with a chain structure composed of vinyl units, and a polyhydric alcohol compound with a chain structure composed of styrene units and vinyl units are obtained.

The polyhydric alcohol compounds with a plurality of kinds of chain structures (polyhydric alcohol compounds with chain structures composed of either styrene units or vinyl units, or both) as described above can be separated using LC such as GPC or reversed phase liquid chromatography (hereinafter referred to as "RPLC"). In this case, the polyhydric alcohol compound with a chain structure composed of styrene units alone and the polyhydric alcohol compound with a chain structure composed of both styrene units and vinyl units can be detected with a UV detector such as an ultraviolet visible light spectroscopic detector by utilizing ultraviolet absorption of the styrene unit. The polyhydric alcohol compound with a chain structure composed of vinyl units alone cannot, however, be detected with a UV detector. In contrast to this, the present invention uses a mass spectrometer, so that polyhydric alcohol compounds with various chain structures obtained by subjecting the copolymer of the conjugated diene compound with the aromatic vinyl compound to ozonolysis can be detected. Besides, when the mass-to-charge ratios of a plurality of components contained in the sample vary, the mass spectrometer can distinguish and detect the plurality of components. Therefore, even though the plurality of kinds of polyhydric alcohol compounds contained in the ozonolysis product of the copolymer are insufficiently separated by LC, they can be individually distinguished and then detected.

In the mass spectrometer, any of a scanning mode for detecting ions while voltage applied to an electrode is continuously changed; a selected ion monitoring (SIM) measurement mode for detecting ions having specific mass-to-charge ratios; and a selected reaction monitoring (SRM) measurement mode for selecting precursor ions having specific mass-to-charge ratios and detecting an ion having a specific mass-to-charge ratio among product ions of the precursor ions may be used, and in particular, in the case of performing quantitative analysis of the polyhydric alcohol compound, SIM measurement or SRM measurement is preferable.

As described above, the plurality of kinds of polyhydric alcohol compounds contained in the ozonolysis product of the copolymer can be accurately detected by combining LC such as GPC or RPLC with a mass spectrometer. However, by using a comprehensive two-dimensional liquid chromatograph (sometimes referred to as "LC x LC", and hereinafter referred to as a comprehensive two-dimensional LC) equipped with two-stage columns including a first column and a second column as LC, the polyhydric alcohol compounds with various chain structures can be more accurately separated, so that the plurality of kinds of polyhydric alcohol compounds can be more accurately detected. With the comprehensive two-dimensional LC, various kinds of components in the sample are first separated in the first column and the eluted component is introduced into a modulator. The modulator repeats an operation in which a solvent (including a component to be analyzed) introduced at every predetermined time interval is stored and the stored solvent is then introduced into the second column. Usually, the first and second columns which have different polarities or different separation modes are used, and a column which exhibits separation behavior different from that of the first column is used as the second column. Thus, even though some of the plurality of kinds of polyhydric alcohol compounds obtained by the ozonolysis of the copolymer and the reduction reaction cannot be sufficiently separated because of the very close elution time in the first column, they can be separated in the second column.

In the case of using the comprehensive two-dimensional LC, a two-dimensional chromatogram may be created with the elution time in the first column and the elution time in the second column as axes and with a signal intensity represented in contour, or a three-dimensional chromatogram may be created with the signal intensity also as an axis, based on detection results with the mass spectrometer. From the shape of the two-dimensional chromatogram or the three-dimensional chromatogram, the chain structure contained in the copolymer can be recognized, which facilitates analysis of the structure or characteristics of the copolymer.

### ADVANTAGEOUS EFFECTS OF INVENTION

According to the present invention, a sample solution containing polyhydric alcohol compounds with various chain structures obtained by subjecting as an analyte a copolymer of a conjugated diene compound with an aromatic vinyl compound to ozonolysis can be separated into a polyhydric alcohol compound with a chain structure composed of styrene units, a polyhydric alcohol compound with a chain structure composed of vinyl units, and a polyhydric alcohol compound with a chain structure composed of styrene units and vinyl units by LC, and the separated products can be analyzed with a mass spectrometer. Therefore, all the polyhydric alcohol compounds with various chain structures contained in the sample solution can be analyzed.

Similarly, according to the method for quantitatively analyzing a polyhydric alcohol compound of the present invention, polyhydric alcohol compounds with various chain structures obtained by subjecting as an analyte a copolymer of a conjugated diene compound with an aromatic vinyl compound to ozonolysis can be separated based on a difference in the chain structure, and the separated products can be analyzed. Therefore, the properties of the copolymer can be evaluated from the analysis results.

### BRIEF DESCRIPTION OF DRAWINGS

Fig. 1(a) to Fig. 1(c) are views for explaining a structure of SBR as an example of an analyte according to the present invention, Fig. 1(a) is a chemical formula of a component constituting a chain structure of SBR; Fig. 1(b) is a schematic view of the chain structure of the SBR; and Fig. 1(c) is a schematic view of a polyhydric alcohol compound obtained by ozonolysis and reduction reaction.
Fig. 2 is a view showing a configuration of major portions of a comprehensive two-dimensional LC/MS.
Fig. 3 is a table showing analytical conditions of the comprehensive two-dimensional LC/MS used in an embodiment of the present invention.
Fig. 4 is a view showing calibration curves of a standard sample.
Fig. 5 is a view showing calibration curves of another standard sample.
Fig. 6 is a view for explaining a method for estimating calibration curves of a polyhydric alcohol compound having an unknown mass-to-charge ratio.
Fig. 7 is a view for explaining a method for estimating calibration curves of another polyhydric alcohol compound having an unknown mass-to-charge ratio.
Fig. 8 is a view for explaining a method for estimating calibration curves of the other polyhydric alcohol compound having an unknown mass-to-charge ratio.
Fig. 9 is a view showing analysis results of the samples using calibration curves.
Fig. 10 is a view showing concentrations of the components obtained from the analysis results of Fig. 9.
Fig. 11A is a two-dimensional chromatogram of a styrene-derived "S2" component, based on detection results of a comprehensive two-dimensional LC/MS of a polyhydric alcohol compound obtained by ozonolysis reaction of sample 1.
Fig. 11B is a two-dimensional chromatogram of a styrene-derived "S2" component, based on detection results of a comprehensive two-dimensional LC/MS of a polyhydric alcohol compound obtained by ozonolysis reaction of sample 2.
Fig. 12 is a table showing contents of components obtained from the two-dimensional chromatogram.
Fig. 13 is a two-dimensional chromatogram in which all components are plotted together, based on detection results of comprehensive two-dimensional LC/MS of a polyhydric alcohol compound obtained by ozonolysis reaction.

### DESCRIPTION OF EMBODIMENTS

A method for analyzing a copolymer, a method for quantitatively analyzing a polyhydric alcohol compound, and a method for separating an ozonolysis product of SBR will be described hereinafter in detail.

### <Analyte>

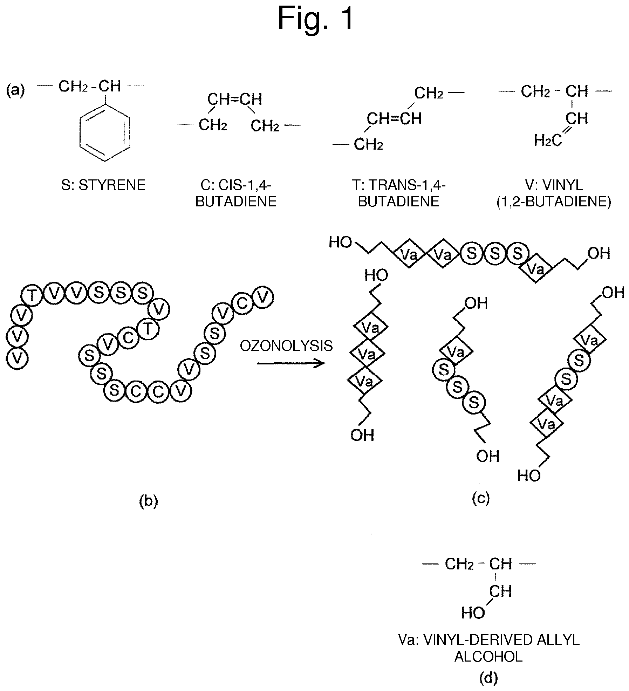

A copolymer of a conjugated diene compound with an aromatic vinyl compound, which is an analyte of the present invention, has a structure in which a structure derived from the conjugated diene compound, a structure derived from the aromatic vinyl, and a structure derived from vinyl are linearly linked, and has a carbon-carbon double bond derived from the conjugated diene compound and the vinyl group. As an example of the copolymer, a styrene-butadiene rubber (hereinafter referred to as SBR), which is a copolymer of 1,3-butadiene with styrene, may be used. The SBR has a chain structure in which a styrene-derived component; cis-1,4-butadiene and trans-1,4-butadiene which are 1,3-butadiene-derived components; and a vinyl group are linearly linked in a large number (see Figs. 1(a) and 1(b)).

### <Ozonolysis>

In the analysis method of the present invention, first, the copolymer of the conjugated diene compound with the aromatic vinyl compound is subjected to ozonolysis, to thereby produce a polyhydric alcohol compound. The ozonolysis method is performed according to the method described in Non-Patent Literatures 2 and 3. According to this method, ozone selectively reacts with a carbon-carbon double bond derived from the conjugated diene compound among the carbon-carbon double bonds contained in the copolymer, so that an ozonide is produced, and the ozonide is then ring-opened by reduction reaction to produce a polyhydric alcohol compound. When the analyte is the SBR, carbon-carbon double bonds derived from butadiene (carbon-carbon double bonds contained in cis-1,4-butadiene and trans-1,4-butadiene) are ring-opened by ozonolysis, so that a polyhydric alcohol compound with a chain structure composed of styrene units, a polyhydric alcohol compound with a chain structure composed of vinyl units, and a polyhydric alcohol compound with a chain structure composed of styrene units and vinyl units are obtained. For example, when the SBR has a chain structure as shown in Fig. 1(b), a polyhydric alcohol compound shown in Fig. 1(c) is obtained by the ozonolysis. In Figs. 1(b) and 1(c), the symbols S, C, T, and V represent structural units of styrene, cis-1,4-butadiene, trans-1,4-butadiene, and vinyl(1,2-butadiene), respectively, and the symbol Va represents a structural unit of allyl alcohol derived from vinyl (1,2-butadiene) (see Fig. 1(d)) after the ozonolysis.

### <Separation of ozonolysis product>

The polyhydric alcohol compounds obtained by ozonolysis reaction are dissolved in a suitable solvent to produce a sample solution, and the sample solution is then introduced into an LC. As the LC, either an LC having one column or an LC having two columns (first column and second column) may be used. In the case of a comprehensive two-dimensional LC having a first column and a second column, separation may be performed in GPC mode in the first column and in reversed phase LC mode in the second column. When the comprehensive two-dimensional LC in which separation is performed in GPC mode in the first column is used, the ozonolysis product of the SBR is analyzed using a gel permeation chromatography, and the past findings from the results of the analysis are applicable.

The comprehensive two-dimensional LC includes a modulator, as well as the first column and the second column. The sample solution introduced in the comprehensive two-dimensional LC is first separated in the first column and the eluted component is introduced into the modulator. The modulator repeats an operation in which the component introduced at every predetermined time interval is stored and the stored component is then introduced into the second column. Usually, the first and second columns are used which have different separation modes. As the first column, a plurality of columns that are connected together can be used. In this case, a plurality of columns containing a stationary phase of the same kind may be connected or a plurality of columns containing a stationary phase of the same kind but having different pore diameters may be connected. Alternatively, a plurality of columns containing different kinds of stationary phases may be connected.

When the analyte is the SBR which is a copolymer of 1,3-butadiene with styrene, the first column is preferably filled with a filler including styrene-divinylbenzene copolymer, polyvinyl alcohol or silica gel, as a base material, and the second column is preferably filled with a filler including silica gel or polymer chemically bonded with octadecylsilyl group, phenyl group, octyl group, or pentafluorophenyl propyl group, as a base material.

In addition, as a mobile phase, one or two or more polar solvents or nonpolar solvents selected from chloroform, tetrahydrofuran, water, acetonitrile, isopropanol, ethyl acetate, acetone, hexane, methanol, and ethanol may be used. By using the first column, the second column, and the mobile phase described above, the polyhydric alcohol compound obtained by ozonolysis of the SBR and reduction reaction can be sufficiently separated.

### <Detector>

The polyhydric alcohol compound separated by LC is subsequently introduced into a detector. A mass spectrometer is used as the detector. **In** addition to the mass spectrometer, a detector such as an ultraviolet visible light spectroscopic detector or a photodiode array detector may be used. **In** the mass spectrometer, any of a scanning mode for detecting ions while voltage applied to an electrode is continuously changed; a selected ion monitoring (SIM) measurement mode for detecting ions having specific mass-to-charge ratios; and a selected reaction monitoring (SRM) measurement mode for selecting precursor ions having specific mass-to-charge ratios and detecting an ion having a specific mass-to-charge ratio among product ions of the precursor ions may be used, and in particular, in the case of performing quantitative analysis of the polyhydric alcohol compound, SIM measurement or SRM measurement is preferable.

### <Analysis method>

In the case of separating the sample solution containing the polyhydric alcohol compound obtained by the ozonolysis reaction with the comprehensive two-dimensional LC, a two-dimensional chromatogram with an elution time in the first column and an elution time in the second column as axes and with a signal intensity of the mass spectrometer represented in contour, or a three-dimensional chromatogram with the elution time in the first column, the elution time in the second column, and the signal intensity of the mass spectrometer as axes can be created based on the detection results with the mass spectrometer. Thus, the chain structure contained in the copolymer can be visually recognized, which facilitates the analysis.

Referring to a calibration curve representing a relationship between the concentration of each kind of the polyhydric alcohol compounds and the ion intensity, the concentration of a polyhydric alcohol compound is calculated from the ion intensity with respect to the polyhydric alcohol compounds in the sample solution detected with the mass spectrometer.

In this case, a calibration curve of a polyhydric alcohol compound having a known mass-to-charge ratio and a known concentration can be created by acquiring a mass chromatogram using results of a standard sample solution containing the polyhydric alcohol compound having the known mass-to-charge ratio and the known concentration detected with a mass spectrometer, and then obtaining an ion intensity from a peak area derived from the known polyhydric alcohol compound appearing on the mass chromatogram.

A calibration curve of a polyhydric alcohol compound having an unknown mass-to-charge ratio may be estimated by using calibration curves created for a plurality of polyhydric alcohol compounds with similar chain structures, the plurality of polyhydric alcohol compounds having known mass-to-charge ratios and known concentrations, to calculate a degree of contribution with respect to ion intensities of components contained in the chain structures.

### <Liquid chromatograph mass spectrometer>

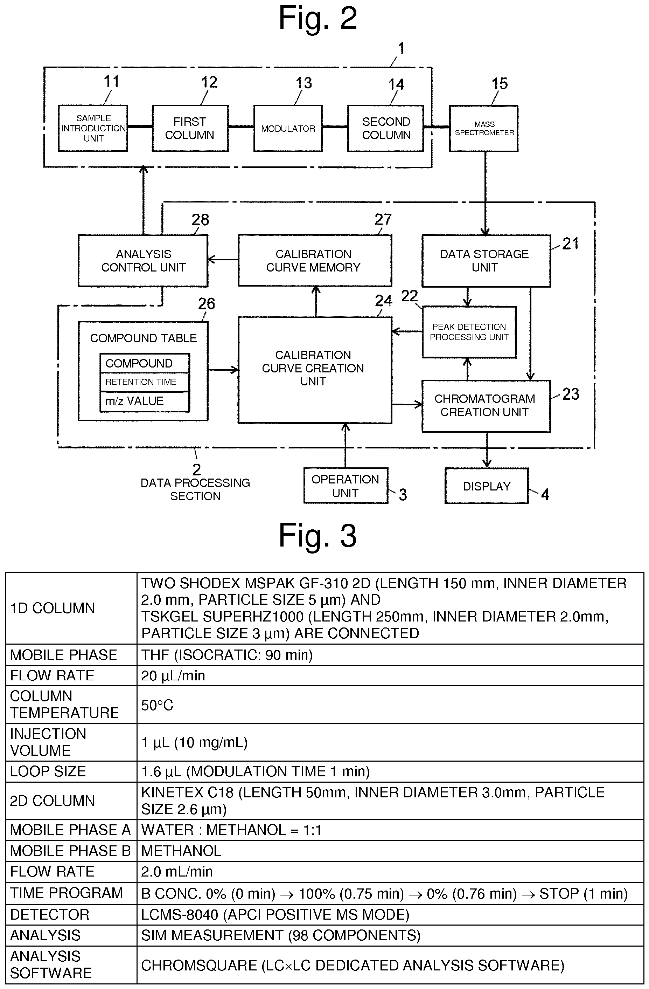

The method for analyzing a copolymer, the method for quantitatively analyzing a polyhydric alcohol compound, and the method for separating an ozonolysis product of SBR is achieved by using a comprehensive two-dimensional LC mass spectrometer (hereinafter referred to as "comprehensive two-dimensional LC/MS") shown in Fig. 2. In the comprehensive two-dimensional LC/MS shown in Fig. 2, an analysis section 1 includes a first column 12, a sample introduction unit 11 that introduces a sample solution into the first column 12, a modulator 13 that stores a component (compound) eluted from the first column 12 at every predetermined time interval, a second column 14 that has a different separation characteristic (typically different polarity) from the first column 12 and that enables high speed separation, and a mass spectrometer 15 that detects the components separated at two-stage columns 12, 14. The mass spectrometer 15 includes, for example, a quadrupole mass filter as a mass analyzer and selectively performs scanning measurement, SIM measurement, and SRM measurement. In the case of requiring more quantitative information, a mass spectrometer including a quadrupole ion trap as a mass analyzer or a time-of-flight mass spectrometer may be used. Alternatively, a hybrid mass spectrometer combined with plurality of kinds of mass analyzers may be used.

The operation of each unit included in the analysis section 1 is controlled by an analysis control unit 28. A data processing section 2 has functions of processing data acquired at the mass spectrometer 15 and of automatically creating a measurement condition file to be used when the analysis control unit 28 performs analysis. More specifically, the data processing section 2 includes function blocks such as a data storage unit 21, a peak detection processing unit 22, a chromatogram creation unit 23, a calibration curve creation unit 24, a compound table 26, a calibration curve memory 27, and the like. The functions of these function blocks will be described later. The blocks as described above function when part of the data processing section 2 or the analysis control unit 28 uses a personal computer as hardware, and dedicated control processing software preinstalled in the personal computer is then executed. In the data processing section 2, an operation unit 3 which is a pointing device such as a keyboard or a mouse and a display 4 are connected. The comprehensive two-dimensional LC/MS of this present example is the same as a conventional comprehensive two-dimensional LC/MS as hardware, but it can substantially have a different configuration by using the control and processing software that is different from conventional one.

### EXAMPLES

Next, a specific example using an ozonolysis product of SBR as the analyte will be described.

### <Creation of calibration curve>

A plurality of kinds of standard samples containing polyhydric alcohol compounds having known mass-to-charge ratios and concentrations were analyzed by comprehensive two-dimensional LC/MS. LCMS-8040 (product name) manufactured by Shimadzu Corporation was used for the analysis, and measurement was performed in APCI positive MS mode. Analytical conditions and analysis software are shown in Fig. 3. In the analysis, GPC was used for primary separation, and RPLC, for secondary separation.

In the standard configuration (e.g., Nexera-e (product name) manufactured by Shimadzu Corporation) of comprehensive two-dimensional LC, a loop of the modulator introduces all the elution solution from the first column to the second column depending on the flow rate and the modulation time. The capacity of the loop (loop size) is, therefore, selected from 20 µL, 50 µL, and 100 µL. In contrast to this, as the analytical conditions listed in Fig. 3, in the case where GPC is used for the primary separation using THF as a mobile phase, the volume of the mobile phase in the primary separation to be introduced for the secondary separation may affect the efficiency of the secondary separation. Then, in this analysis, when a small capacity loop (1.6 µL) was installed in the modulator to attempt to reduce solvent effects, the volume of the elution solution introduced from the first column to the second column decreased, but the separation in the second column was improved.

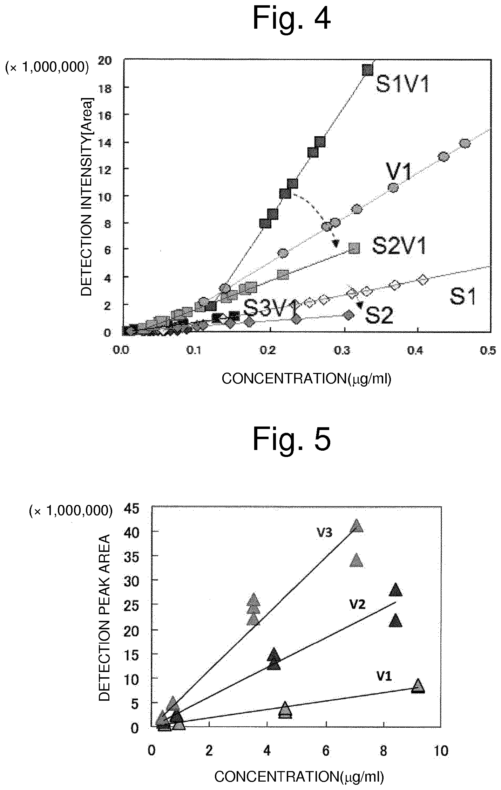

A correlation plot (calibration curve) between concentration and ion intensity of the polyhydric alcohol compound contained in each of the standard samples was created based on the detection results with the mass spectrometer. The created correlation plots are shown in Figs. 4 and 5. **In** Figs. 4 and 5, the symbols "S1V1", "V1", "S2", and the like represent the number of vinyl-derived structures and the number of styrene-derived structures in the polyhydric alcohol compound which shows the calibration curve. For example, the symbol "S1V1" indicates that the polyhydric alcohol compound has one vinyl (1,2-butadiene)-derived structure and one styrene-derived structure. As apparent from Figs. 4 and 5, the calibration curve varies depending on the number of styrene-derived structures and the number of vinyl-derived structures contained in the polyhydric alcohol compound.

As the standard sample, a commercially available sample may be used, or a sample may also be used which is obtained by collecting a compound separated from an ozonolysis product of SBR and calculating its purity by NMR or the like.

### <Estimation of calibration curve>

In the case of an SBR having an unknown structure, quantitative analysis of all the polyhydric alcohol compounds cannot be performed with the calibration curves of the polyhydric alcohol compounds shown in Figs. 4 and 5 alone because various kinds of polyhydric alcohol compounds are obtained by the ozonolysis and the reduction reaction. Therefore, a calibration curve of a polyhydric alcohol compound having an unknown mass-to-charge ratio was estimated from the calibration curves created for the standard samples described above.

Specifically, for a chain component Sn of styrene alone, when the number of chains of styrene increased by one, the change amounts of the inclinations of the calibration curves S1 to S3 were calculated to estimate an inclination of a calibration curve for the chain component Sn (n ≥ 4). For a chain component (Vm) of vinyl-derived allyl alcohol alone, an inclination of a calibration curve was estimated in the same manner as above.

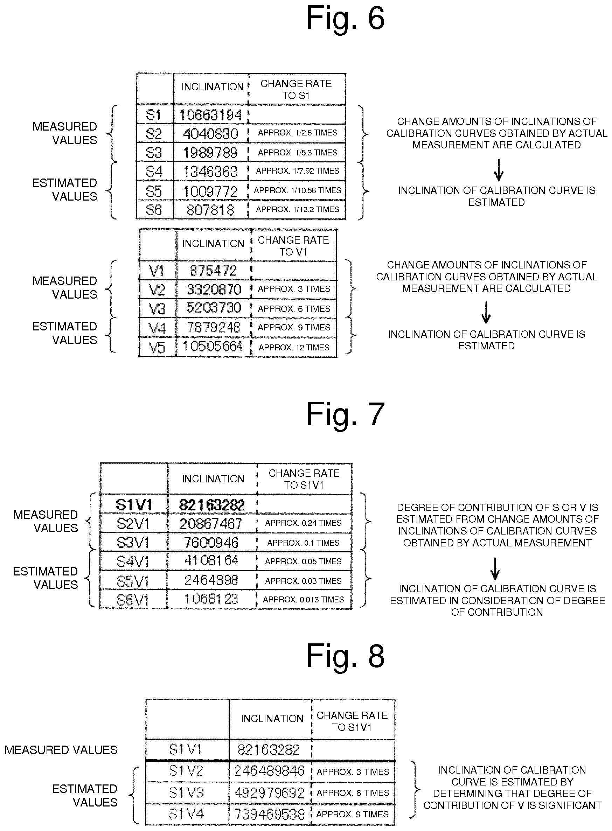

For a chain component (SnVm) of styrene and vinyl-derived allyl alcohol, the degree of contribution of the styrene and the vinyl-derived allyl alcohol with respect to the inclination of the calibration curve was obtained from the change amount of the inclination when the number of chains of styrene was fixed and the number of chains of vinyl-derived allyl alcohol was changed, and the change amount of the inclination when the number of chains of vinyl-derived allyl alcohol was fixed and the number of chains of styrene was changed, and the inclination of the calibration curve of the chain component SnVm was then estimated from the degree of contribution and the number of chains of the styrene and the vinyl-derived allyl alcohol. Figs. 6 to 8 show the inclinations of the calibration curves of the chain components obtained from the actual measurement results and the inclinations of the calibration curves estimated by the above-mentioned method.

### <Analysis of sample using calibration curve>

Using the calibration curves of the polyhydric alcohol compound obtained by the above-mentioned method, chain structures of four SBRs (SBR-A (styrene/vinyl=15.5/46.9 (mol%)), SBR-B (styrene/vinyl=15.5/48.2 (mol%)), SBR-C (styrene/vinyl=15.1/49.8 (mol%)), and SBR-D (styrene/vinyl=15.1/49.6 (mol%))) having known molar ratios of the styrene component/vinyl component were analyzed. The equipment used for the analysis and the analytical conditions are the same as those used for the creation of calibration curves.

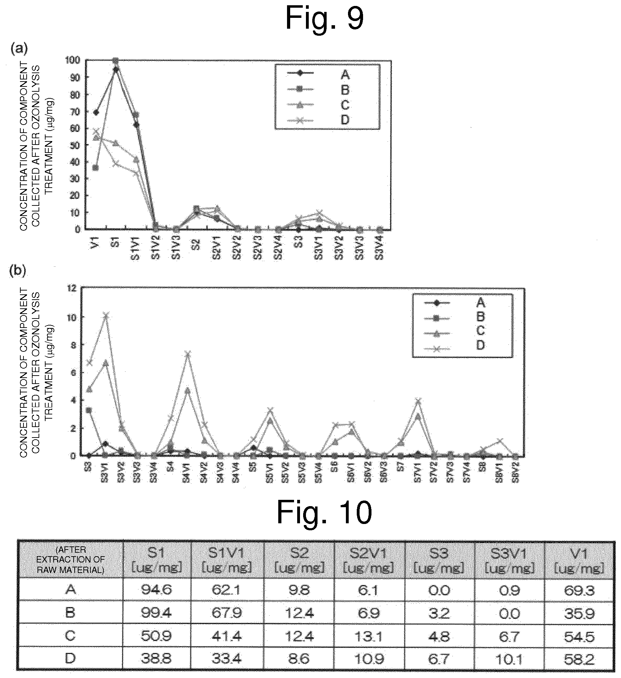

The analysis results are shown in Figs. 9 and 10. Fig. 9 is a graph showing the amount of the chain components in the components collected after the ozonolysis treatment of the four kinds of SBRs (SBR-A to SBR-D) with an abscissa representing kinds of chain components and an ordinate representing the amounts, and Fig. 10 is a table listing the amounts of the representative chain components. As apparent from Figs. 9 and 10, the components collected after the ozonolysis of SBR-A and SBR-B contain excessively large amounts of S1, while the components collected after the ozonolysis of SBR-C and SBR-D contain not only S1 but S3 (S3, S3Vm), S4 (S4Vm) and the like, and also contain a large amount of chain components with 3 or more S. Accordingly, it could be confirmed that even in the case where the amount ratio of styrene is similar to that of vinyl, their chain structures were different.

### <Creation of two-dimensional chromatogram>

Next, two kinds of commercially available styrene-butadiene copolymers (sample 1: SBR-E (styrene/vinyl=12.1/56.1 (mol%)), sample 2: SBR-F (styrene/vinyl=13.8/8.5 (mol%))) as samples were subjected to ozonolysis reaction to produce a polyhydric alcohol compound, and the polyhydric alcohol compound thus produced was analyzed by comprehensive two-dimensional LC/MS. The equipment used for the analysis and the analytical conditions are the same as those used for creation of the calibration curve.

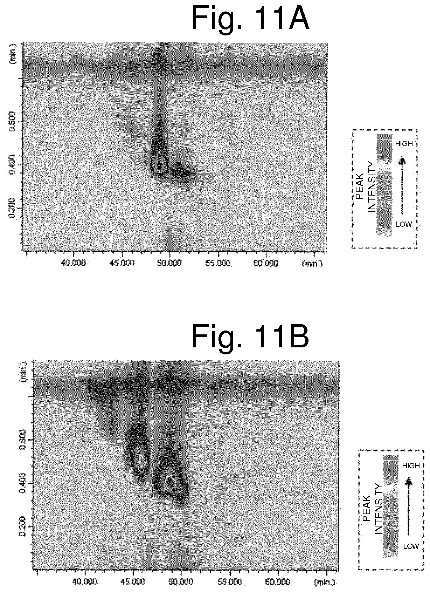

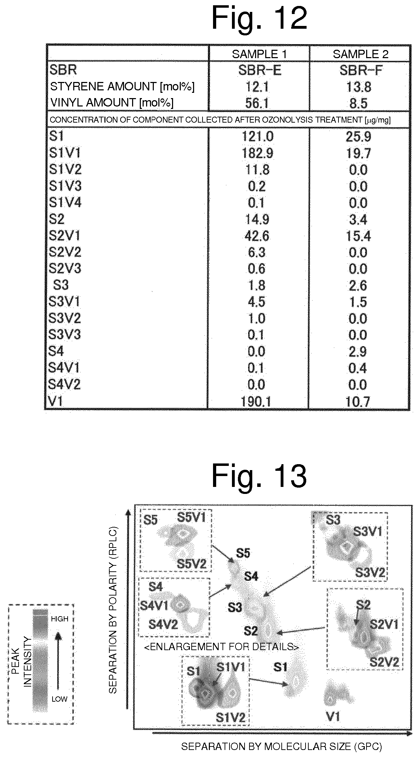

The detection results with the mass spectrometer was analyzed using the analysis software Chrom Square shown in the table in Fig. 3, and a two-dimensional chromatogram with an abscissa representing the elution time in the first column, an ordinate representing the elution time in the second column, and a contour representing the signal intensity was created. As an example of the two-dimensional chromatogram created for the ozonolysis products of the samples 1 and 2, two-dimensional chromatograms of the polyhydric alcohol compound ("S2" component) in which the number of styrenes is 2 are shown in Figs. 11A and 11B. Further, the contents of the polyhydric alcohol compounds in the samples 1 and 2 calculated from the two-dimensional chromatogram are shown in Fig. 12. Thus, in the present example, it was possible to compare the chain structures of the two samples having different contents of styrene and vinyl.

Although Figs. 11A and 11B show the two-dimensional chromatograms of the "S2" component alone, it is possible to plot the detection results of all the components obtained by the ozonolysis together in a two-dimensional chromatogram. Thus, using the comprehensive two-dimensional LC, even the components that are difficult to be separated in a single analysis of individual dimensions like S1V1 and S1V2 can be separated, so that the difference in the chain structures contained in the copolymer can be visually recognized by displaying the detection results in the two-dimensional chromatogram.

The example using the comprehensive two-dimensional LC/MS is described herein, but LC such as reversed phase LC may be used instead of the comprehensive two-dimensional LC. In this case, even though the separation by LC is insufficient, a plurality of components contained in the sample containing the ozonolysis product of the copolymer of the conjugated diene monomer with the aromatic vinyl monomer can be distinguishingly detected if their mass-to-charge ratios are different. Therefore, the same analysis as the comprehensive two-dimensional LC/MS is of course possible.

## Claims

1. A method for analyzing a copolymer, the method comprising:
a) subjecting a copolymer of a conjugated diene compound with an aromatic vinyl compound to ozonolysis to produce a plurality of kinds of polyhydric alcohol compounds;
b) separating a sample solution containing the plurality of kinds of polyhydric alcohol compounds with a comprehensive two-dimensional liquid chromatograph equipped with a first column (12) and a second column (14); and
c) detecting the separated sample with a detector,
wherein:
the detector is a mass spectrometer (15); and
the first column (12) and the second column (14) have different polarities or different separation modes; and wherein the method comprises:
referring to a calibration curve representing a relationship between a concentration of each kind of the polyhydric alcohol compounds and an ion intensity of the detector; and
calculating a concentration of a polyhydric alcohol compound from the ion intensity with respect to the polyhydric alcohol compounds in the sample solution detected with the detector.

2. The method for analyzing a copolymer according to claim 1, comprising creating a two-dimensional chromatogram with an elution time in the first column and an elution time in the second column as axes and with a signal intensity of the mass spectrometer represented in contour, or a three-dimensional chromatogram with the elution time in the first column, the elution time in the second column, and the signal intensity of the mass spectrometer as axes, based on detection results with the mass spectrometer.

3. The method for analyzing a copolymer according to claim 1, wherein the conjugated diene compound is 1,3-butadiene and the aromatic vinyl compound is styrene.

4. The method for analyzing a copolymer according to claim 3, wherein the first column (12) is filled with a filler including styrene-divinylbenzene copolymer, a polyvinyl alcohol or silica gel, as a base material.

5. The method for analyzing a copolymer according to claim 3, wherein the second column (14) is filled with a filler including silica gel or polymer chemically bonded with octadecylsilyl group, phenyl group, octyl group, or pentafluorophenyl propyl group, as a base material.

6. The method for analyzing a copolymer according to claim 1, wherein a sample solution is introduced into a comprehensive two-dimensional liquid chromatograph together with a mobile phase containing one or two or more solvents selected from chloroform, tetrahydrofuran, water, acetonitrile, isopropanol, ethyl acetate, acetone, hexane, methanol, and ethanol in the separating.

7. The method for analyzing a copolymer according to claim 1, wherein a calibration curve of a polyhydric alcohol compound having a known mass-to-charge ratio and a known concentration is created by acquiring a mass chromatogram using results of a standard sample solution containing the polyhydric alcohol compound having the known mass-to-charge ratio and the known concentration detected with the mass spectrometer, and then obtaining an ion intensity from a peak area derived from the known polyhydric alcohol compound appearing on the mass chromatogram.

8. The method for analyzing a copolymer according to claim 7, wherein
a calibration curve of a polyhydric alcohol compound having an unknown mass-to-charge ratio is estimated by using calibration curves created for a plurality of polyhydric alcohol compounds with similar chain structures, the plurality of polyhydric alcohol compounds having known mass-to-charge ratios and known concentrations, to calculate a degree of contribution with respect to ion intensities of components contained in the chain structures.

9. The method for analyzing a copolymer according to claim 1, the method comprising:
subjecting a styrene-butadiene rubber to ozonolysis and then reduction to produce a polyhydric alcohol compound with a chain structure composed of a component derived from styrene, a polyhydric alcohol compound with a chain structure composed of a component derived from vinyl, and a polyhydric alcohol compound with a chain structure composed of a component derived from styrene and a component derived from vinyl.

10. The method for analyzing a copolymer according to claim 1, wherein the separation is performed in GPC mode in the first column and in reversed phase LC mode in the second column.

11. A data processor that processes data obtained by separating with a comprehensive two-dimensional liquid chromatograph equipped with a first column (12) and a second column (14) a sample solution containing plurality of kinds of polyhydric alcohol compounds obtained by subjecting a copolymer of a conjugated diene compound with an aromatic vinyl compound to ozonolysis, and then detecting the separated products with a detector,
the data processor comprising:
a storage unit that stores a calibration curve representing a relationship between a concentration of each kind of the polyhydric alcohol compounds and an ion intensity of the detector; and
a concentration calculation means that calculates a concentration of each of the polyhydric alcohol compounds in the sample solution from the data, with reference to the calibration curve stored in the storage unit,
wherein,
the detector is a mass spectrometer (15); and
the first column (12) and the second column (14) have different polarities or different separation modes.

## Patentansprüche

1. Verfahren zum Analysieren eines Copolymers, wobei das Verfahren Folgendes umfasst:
a) das Aussetzen eines Copolymers einer konjugierten Dien-Verbindung mit einer aromatischen Vinyl-Verbindung gegenüber Ozonolyse, um eine Vielzahl von Arten von mehrwertigen Alkohol-Verbindungen zu erzeugen,
b) das Trennen einer Probenlösung, welche die Vielzahl von Arten von mehrwertigen Alkohol-Verbindungen enthält, mit einem umfassenden zweidimensionalen Flüssigchromatographen, der mit einer ersten Säule (12) und einer zweiten Säule (14) ausgestattet ist, und
c) das Detektieren der getrennten Probe mit einem Detektor;
wobei:
der Detektor ein Massenspektrometer (15) ist und
die erste Säule (12) und die zweite Säule (14) verschiedene Polaritäten oder verschiedene Trennmodi aufweisen; und
wobei das Verfahren Folgendes umfasst:
das Bezugnehmen auf eine Kalibrierkurve, die eine Beziehung zwischen der Konzentration jeder Art der mehrwertigen Alkohol-Verbindungen und der Ionenintensität des Detektors darstellt; und
das Berechnen der Konzentration einer mehrwertigen Alkohol-Verbindung aus der Ionenintensität in Bezug auf die mehrwertigen Alkohol-Verbindungen in der Probenlösung, die mit dem Detektor detektiert wurde.

2. Verfahren zum Analysieren eines Copolymers nach Anspruch 1, welches das Erstellen eines zweidimensionalen Chromatogramms mit der Elutionszeit in der ersten Säule und der Elutionszeit in der zweiten Säule als Achsen und mit der Signalintensität des Massenspektrometers, die in Umrissen dargestellt ist, oder eines dreidimensionalen Chromatogramms mit der Elutionszeit in der ersten Säule, der Elutionszeit in der zweiten Säule und der Signalintensität des Massenspektrometers als Achsen, basierend auf den Detektionsergebnissen mit dem Massenspektrometer umfasst.

3. Verfahren zum Analysieren eines Copolymers nach Anspruch 1, wobei die konjugierte Dien-Verbindung 1,3-Butadien ist und die aromatische Vinyl-Verbindung Styrol ist.

4. Verfahren zum Analysieren eines Copolymers nach Anspruch 3, wobei die erste Säule (12) mit einem Füllstoff, der Sytrol-Divinylbenzol-Copolymer, einen Polyvinylalkohol oder Kieselgel umfasst, als Basismaterial gefüllt ist.

5. Verfahren zum Analysieren eines Copolymers nach Anspruch 3, wobei die zweite Säule (14) mit einem Füllstoff, der Kieselgel oder ein Polymer umfasst, das mit einer Octadecylsilylgruppe, einer Phenylgruppe, einer Octylgruppe oder einer Pentafluorphenylpropylgruppe chemisch verbunden ist, als Basismaterial gefüllt ist.

6. Verfahren zum Analysieren eines Copolymers nach Anspruch 1, wobei die Probenlösung zusammen mit einer mobilen Phase, die ein oder oder zwei oder mehr Lösungsmittel enthält, die aus Chloroform, Tetrahydrofuran, Wasser, Acetonitril, Isopropanol, Ethylacetat, Aceton, Hexan, Methanol und Ethanol ausgewählt sind, in dem Trennschritt in einen umfassenden zweidimensionalen Flüssigchromatographen eingebracht werden.

7. Verfahren zum Analysieren eines Copolymers nach Anspruch 1, wobei eine Kalibrierkurve einer mehrwertigen Alkohol-Verbindung, die ein bekanntes Masse-Ladungs-Verhältnis und eine bekannte Konzentration aufweist, erstellt wird, indem ein Massenchromatogramm unter Verwendung von Ergebnissen einer Standard-Probenlösung erhalten wird, welche die mehrwertige Alkohol-Verbindung mit dem bekannten Masse-Ladungs-Verhältnis und der bekannten Konzentration enthält, das mit dem Massenspektrometer detektiert wurde, und dann die Ionenintensität von der Peakfläche, die der bekannten mehrwertigen Alkohol-Verbindung entspricht, die auf dem Massenchromatogramm aufscheint, erhalten wird.

8. Verfahren zum Analysieren eines Copolymers nach Anspruch 7, wobei eine Kalibrierkurve einer mehrwertigen Alkohol-Verbindung, die ein unbekanntes Masse-Ladungs-Verhältnis aufweist, unter Verwendung von Kalibrierkurven abgeschätzt wird, die für eine Vielzahl von mehrwertigen Alkohol-Verbindungen mit ähnlichen Kettenstrukturen erstellt wurden, wobei die Vielzahl von mehrwertigen Alkohol-Verbindungen bekannte Masse-Ladungs-Verhältnisse und bekannte Konzentrationen aufweisen, um den Beitragsgrad bezogen auf lonenintensitäten von in den Kettenstrukturen enthaltenen Komponenten zu berechnen.

9. Verfahren zum Analysieren eines Copolymers nach Anspruch 1, wobei das Verfahren Folgendes umfasst:
das Aussetzen eines Styrol-Butadien-Kautschuks gegenüber Ozonolyse und anschließender Reduktion, um eine mehrwertige Alkohol-Verbindung mit einer Kettenstruktur, die aus einer Komponente besteht, die von Styrol abgeleitet ist, eine mehrwertige Alkohol-Verbindung mit einer Kettenstruktur, die aus einer Komponente besteht, die von Vinyl abgeleitet ist, und eine mehrwertige Alkohol-Verbindung mit einer Kettenstruktur, die aus einer Komponente, die von Styrol abgeleitet ist, und einer Komponente besteht, die von Vinyl abgeleitet ist, zu erzeugen.

10. Verfahren zur Analyse eines Copolymers nach Anspruch 1, wobei die Trennung im GPC-Modus in der ersten Säule und im Umkehrphasen-LC-Modus in der zweiten Säule durchgeführt wird.

11. Datenprozessor, der Daten verarbeitet, die durch das Trennen einer Probenlösung, die eine Vielzahl von Arten von mehrwertigen Alkohol-Verbindungen enthält, die durch Aussetzen eines Copolymers einer konjugierten Dien-Verbindung mit einer aromatischen Vinyl-Verbindung gegenüber Ozonolyse erhalten wurde, mit einem umfassenden zweidimensionalen Flüssigchromatographen, der mit einer ersten Säule (12) und einer zweiten Säule (14) ausgestattet ist, und anschließendes Detektieren der getrennten Produkte mit einem Detektor erhalten wurden;
wobei der Datenprozessor Folgendes umfasst:
eine Speichereinheit, die eine Kalibrierkurve speichert, welche die Beziehung zwischen der Konzentration jeder Art der mehrwertigen Alkohol-Verbindungen und der Ionenintensität des Detektors darstellt, und
ein Konzentrationsberechnungsmittel, das die Konzentration jeder der mehrwertigen Alkohol-Verbindungen in der Probenlösung ausgehend von den Daten bezogen auf die in der Speichereinheit gespeicherten Kalibrierkurve berechnet;
wobei
der Detektor ein Massenspektrometer (15) ist und
die erste Säule (12) und die zweite Säule (14) unterschiedliche Polaritäten oder unterschiedliche Trennmodi aufweisen.

## Revendications

1. Procédé d'analyse d'un copolymère, le procédé comprenant les étapes consistant à :
a) soumettre un copolymère d'un composé de diène conjugué avec un composé de vinyle aromatique à une ozonolyse pour produire une pluralité de types de composés d'alcool polyhydrique ;
b) séparer une solution d'échantillon contenant la pluralité de types de composés d'alcool polyhydrique en utilisant un chromatographe liquide bidimensionnel complet équipé d'une première colonne (12) et d'une seconde colonne (14) ; et
c) détecter l'échantillon séparé à l'aide d'un détecteur,
dans lequel :
le détecteur est un spectromètre de masse (15) ; et
la première colonne (12) et la seconde colonne (14) présentent des polarités différentes ou des modes de séparation différents ; et dans lequel le procédé comprend les étapes consistant à :
se référer à une courbe d'étalonnage représentant une relation entre une concentration de chaque type des composés d'alcool polyhydrique et une intensité ionique du détecteur ; et
calculer une concentration d'un composé d'alcool polyhydrique à partir de l'intensité ionique par rapport aux composés d'alcool polyhydrique dans la solution d'échantillon détectée à l'aide du détecteur.

2. Procédé d'analyse d'un copolymère selon la revendication **1,** comprenant une création d'un chromatogramme bidimensionnel avec un temps d'élution dans la première colonne et un temps d'élution dans la seconde colonne en tant qu'axes et avec une intensité de signal du spectromètre de masse représentée en contour, ou d'un chromatogramme tridimensionnel avec le temps d'élution dans la première colonne, le temps d'élution dans la seconde colonne et l'intensité de signal du spectromètre de masse en tant qu'axes, sur la base des résultats de détection à l'aide du spectromètre de masse.

3. Procédé d'analyse d'un copolymère selon la revendication 1, dans lequel le composé de diène conjugué est le 1,3-butadiène et le composé de vinyle aromatique est le styrène.

4. Procédé d'analyse d'un copolymère selon la revendication 3, dans lequel la première colonne (12) est remplie d'une charge incluant un copolymère de styrène-divinylbenzène, un alcool polyvinylique ou un gel de silice, en tant que matériau de base.

5. Procédé pour analyser un copolymère selon la revendication 3, dans lequel la seconde colonne (14) est remplie d'une charge incluant un gel de silice ou un polymère lié chimiquement à un groupe octadécylsilyle, un groupe phényle, un groupe octyle ou un groupe pentafluorophénylpropyle, en tant que matériau de base.

6. Procédé d'analyse d'un copolymère selon la revendication 1, dans lequel une solution d'échantillon est introduite dans un chromatographe liquide bidimensionnel complet avec une phase mobile contenant un ou deux solvants ou plus choisis parmi le chloroforme, le tétrahydrofuranne, l'eau, l'acétonitrile, l'isopropanol, l'acétate d'éthyle, l'acétone, l'hexane, le méthanol et l'éthanol dans la séparation.

7. Procédé d'analyse d'un copolymère selon la revendication 1, dans lequel une courbe d'étalonnage d'un composé d'alcool polyhydrique présentant un rapport masse sur charge connu et une concentration connue est créée en acquérant un chromatogramme de masse en utilisant les résultats d'une solution d'échantillon standard contenant le composé d'alcool polyhydrique présentant le rapport masse sur charge connu et la concentration connue détectés à l'aide du spectromètre de masse, puis en obtenant une intensité ionique à partir d'une zone de pic dérivée du composé d'alcool polyhydrique connu apparaissant sur le chromatogramme de masse.

8. Procédé d'analyse d'un copolymère selon la revendication 7, dans lequel une courbe d'étalonnage d'un composé d'alcool polyhydrique présentant un rapport masse sur charge inconnu est estimée en utilisant des courbes d'étalonnage créées pour une pluralité de composés d'alcool polyhydrique présentant des structures de chaîne similaires, la pluralité de composés d'alcool polyhydrique présentant des rapports masse sur charge connus et des concentrations connues, pour calculer un degré de contribution par rapport aux intensités ioniques de composants contenus dans les structures de chaîne.

9. Procédé d'analyse d'un copolymère selon la revendication 1, le procédé comprenant en outre l'étape consistant à :
soumettre un caoutchouc de styrène-butadiène à une ozonolyse, puis à une réduction pour produire un composé d'alcool polyhydrique avec une structure de chaîne composée d'un composant dérivé du styrène, un composé d'alcool polyhydrique avec une structure de chaîne composée d'un composant dérivé du vinyle, et un composé d'alcool polyhydrique avec une structure de chaîne composée d'un composant dérivé du styrène et d'un composant dérivé du vinyle.

10. Procédé pour analyser un copolymère selon la revendication 1, dans lequel la séparation est effectuée en mode GPC dans la première colonne et en mode LC à phase inversée dans la seconde colonne.

11. Processeur de données qui traite des données obtenues en séparant, à l'aide d'un chromatographe en phase liquide bidimensionnel complet équipé d'une première colonne (12) et d'une seconde colonne (14), une solution d'échantillon contenant une pluralité de types de composés d'alcool polyhydrique obtenus en soumettant un copolymère d'un composé de diène conjugué avec un composé vinylique aromatique à une ozonolyse, puis en détectant les produits séparés à l'aide d'un détecteur,
le dispositif de traitement de données comprenant :
une unité de stockage qui stocke une courbe d'étalonnage représentant une relation entre une concentration de chaque type de composés d'alcool polyhydrique et une intensité ionique du détecteur ; et
un moyen de calcul de concentration qui calcule une concentration de chacun des composés d'alcool polyhydrique dans la solution d'échantillon à partir des données, en référence à la courbe d'étalonnage stockée dans l'unité de stockage,
dans lequel,
le détecteur est un spectromètre de masse (15) ; et
la première colonne (12) et la seconde colonne (14) présentent des polarités différentes ou des modes de séparation différents.
